# EUROPEAN PATENT SPECIFICATION

(11) **EP 1 957 049 B1**
(45) Date of publication and mention of the grant of the patent: **19.08.2009**
(21) Application number: 06818790.5
(22) Date of filing: 24.11.2006
(51) Int. Cl.: A61K 9/16, A61K 9/50, A61K 9/00

(54) **GRANULAR, GASTRO-RESISTANT PRODUCT BASED ON NIACIN OR DERIVATES THEREOF AND PROCESS FOR THE PRODUCTION OF SUCH A PRODUCT**
GRANULATFÖRMIGES MAGENRESISTENTES PRODUKT AUF BASIS VON NIACIN ODER SEINEN DERIVATEN UND VERFAHREN ZUR HERSTELLUNG EINES SOLCHEN PRODUKTS
PRODUIT GRANULAIRE ET GASTRO-RESISTANT À BASE DE NIACINE OU DE SES DERIVÉS ET PROCÉDÉ DE FABRICATION D'UN TEL PRODUIT

(30) Priority: 29.11.2005 IT PD20050347
(43) Date of publication of application: 20.08.2008
(73) Proprietor: SILA S.r.l., 30030 Noale (VE) (IT)
(72) Inventor: LORENZON, Maurizio, I-30033 Noale (IT)
(74) Representative: Susanetto, Carlo
(86) International application number: PCT/EP2006/011271
(87) International publication number: WO 2007/062779

(56) References cited:
- GB-A- 285 091
- US-A- 3 037 911
- US-A- 3 080 293
- US-A1- 2004 185 170
- US-B1- 6 485 742

## Description

### Technical Field

The present invention relates to a granular, gastro-resistant product based on niacin or derivatives thereof according to the features set out in the precharacterising clause of main claim 1. It is also directed towards a process for the production of such a product having the features stated in the precharacterising clause of independent claim 11.

### Background Art

Niacin, which is also known as vitamin B₃ or vitamin PP, is a substance which has very important metabolic functions in humans and animals, being a precursor of the coenzymes NAD and NADP which, in turn, are involved in the oxidation-reduction reactions (for example in the Krebs cycle) from which an organism obtains the energy it requires for its activities.

Strictly speaking, niacin corresponds to nicotinic acid, the structural formula of which is shown in (I), but the term is often used by analogy also to denote the amide of nicotinic acid, nicotinamide, the structural formula of which is shown in (II).

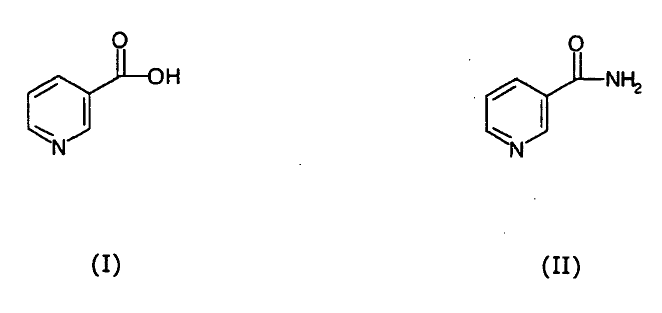

A deficiency of niacin in the organism results in an overall slowing of metabolism which is manifested, in the most serious cases, in the disorder known as pellagra. Niacin furthermore exhibits vasodilatory properties, for which reason it may be used for the therapeutic treatment of arterial hypertension, and is furthermore known to be effective in the control of high levels of cholesterol in the blood (hypercholesterolaemia). In particular, niacin is used for reducing the concentration of low-density cholesterol (LDL-C), known as "bad cholesterol" due to its direct involvement in cardiac disease.

The niacin required for correct functioning of the organism is largely obtained from outside the body, already in the form of NAD or NADP, in particular by intake of foods of animal origin, but if necessary it may also be taken as such, either in the form of nicotinic acid or in the form of nicotinamide.

It is, however, known that taking relatively large quantities of niacin may bring about the appearance of severe and unwanted skin redness similar to sunburn (a phenomenon known as "flushing") which is often accompanied by pruritus, gastric disorders and a reduction in arterial pressure. This series of contraindications usually has very severe impact on individuals, such that, according to recent clinical studies, a very high proportion of patients, estimated to be between 10% and 50%, would give up treatment precisely because of these disadvantages.

In order to limit the contraindications associated with the administration of niacin, in particular the phenomenon of flushing, various solutions have been developed which aim to slow the release of niacin in the organism, in particular its release in the stomach. A first example of such solutions involves dispersing an appropriate quantity of niacin in a matrix of excipient substances, while a second known solution, described for example in United States patent 4,868,180, proposes encapsulating the niacin in a coating based on cellulosic substances.

Both US patents n. 3080293 and n. 3037911, disclose a process for incorporating niacin in a saturated fatty acid matrix, in particular in a stearic acid matrix, in order to improve the disagreeable taste of the niacin as such.

However, these solutions do not satisfactorily overcome the drawbacks encountered, for which reason a need subsists in this technical field to provide a niacin-based product which exhibits reduced side-effects, in particular which significantly reduces the phenomenon of flushing.

### Disclosure of the invention

The problem underlying the present invention is that of providing a granular, gastro-resistant product based on niacin or derivatives thereof and of providing a process for the production of such a product, these being structurally and functionally designed to overcome the above-stated shortcomings of the cited prior art.

In the context of this problem, a first object of the invention is furthermore to provide a granular product based on nicotinic acid in a form usable in the most varied applications in the field of human nutrition.

A second object is to provide a granular product with an elevated nicotinic acid content.

Said problem is solved and said objects are achieved by the present invention by means of a granular product and a process for the production of such a product in accordance with the following claims.

The features and advantages of the invention will emerge more clearly from the following detailed description in conjunction with as preferred embodiment thereof.

The product of the present invention is produced from niacin or derivatives thereof in granular form, which, in a first phase of the process, is uniformly mixed with a matrix of appropriate composition defined in greater detail below.

The mixing operation is carried out at a temperature such as to cause a large proportion of the components of the matrix to melt, but, at the same time, such as not to cause thermal degradation or impairment of the substances present in the mixture. This temperature is less than 120°C and, preferably, is between 60°C and 75°C, and, still more preferably, is around 70°C.

Mixing is carried out with a level of agitation and for a period of time which are sufficient to make the mixture as uniform and homogeneous as possible.

The granules of nicotinic acid, preferably in pure form, have a size of between 0.1 and 1.5 mm, which is also selected as a function of the desired mean size and size distribution for the finished product. The nicotinic acid granules are preferably between 0.1 and 0.5 mm in size.

According to a first aspect of the present invention, the matrix used in the mixture has a total content of long-chain C16-C22 saturated fatty acids of between 40% and 95%, relative to the matrix. In particular, it comprises a weight fraction of saturated C18 (stearic acid) of between 40% and 95%, preferably between 60% and 80%.

In this context, the total content of fatty acids is calculated taking account both of the presence of fatty acids as such and of the presence thereof in ester or salt form. In particular, the matrix comprises a component based on glycerides, which includes the above-stated C16-C22 fatty acids, and may comprise not only monoglycerides, but also diglycerides and triglycerides. Preferably, the fatty acid component associated with glycerides originates from triglycerides derived from vegetable oils (such as for example palm oil and sunflower oil) or from animal fats (such as for example lard or suet) with an elevated content of long-chain fatty acids. The triglycerides derived from these sources are appropriately hydrogenated to remove any double bonds optionally present in the chain, so as to give rise to saturated fatty acids in the above-stated percentages.

The matrix may optionally also comprises triglycerides having C12-C14 medium to short chain fatty acids, such as for example those derived from coconut oil and/or from soya oil, or, on the contrary, more complex longer chain aliphatic substances, such as for example waxes.

According to a second aspect of the invention, the matrix also contains mineral agents, such as for example calcium carbonate CaCO₃ or, preferably, calcium sulfate dihydrate CaSO₄*2(H₂O).

The mineral agent is present in the matrix in a weight fraction of between 2% and 20%. The presence of said mineral agent makes it possible to impart the correct consistency to the matrix either in the molten state or in the solid state and assists in ensuring that the finished product exhibits correct release kinetics of nicotinic acid in the organism.

The matrix furthermore contains an effective quantity of emulsifying agent, such as to make the matrix as uniform and homogeneous as possible in the molten state.

Suitable emulsifying agents comprise salts and esters of fatty acids, lecithins, esters of mono- and diglycerides, sugar esters of fatty acids, sorbitan-based esters and others.

Of course, should the emulsifying agent comprise a salt or an ester of a C16-C22 saturated fatty acid, this quantity, appropriately broken down, will be included in the overall calculation of the C16-C22 saturated fatty acids, with the aim of obtaining the above-stated weight fractions.

The matrix may furthermore contain further additives, such as for example natural flavours, in a variable concentration of between 0% and 2%.

The weight fraction of nicotinic acid granules mixed with the matrix is the highest fraction which permits the finished granular product to maintain its characteristics of slow release into the organism without giving rise to significant flushing phenomena. In particular, it has surprisingly been found that, by using a matrix produced according to the present invention, it is possible to obtain finished granular products complying with the above-stated requirements which comprise a weight fraction of nicotinic acid of approximately 70%.

However, to ensure a greater safety margin, it is preferred to add nicotinic acid granules to the matrix in a quantity such as to obtain a weight fraction of approximately 50%.

Once the desired level of homogeneity has been obtained, the mixture is supplied at high pressure to a spraying chamber where, by passing through an appropriately shaped jet, it is dispersed into a plurality of particles. The temperature in the spraying chamber is kept at values very much below the solidification temperature of the matrix, preferably of below -5°C, still more preferably at a temperature of between -20°C and -25°C, such that, during the particles' brief residence time in the air, the liquid component thereof is conveniently able to solidify.

The size of the granules obtained from solidification of the biphasic particles sprayed into the chamber is a function of the shape and diameter of the spray jet, together with other parameters such as the velocity at which the mixture is sprayed, the viscosity thereof etc.. Said parameters are appropriately adjusted such that the majority of granules are of a size within the desired range of between 0.15 and 2 mm, preferably of between 0.25 and 0.8 mm.

In any event, the product obtained is however subjected to a screening process which makes it possible to select the grain size which fulfils the required standards.

The finished product obtained exhibits a granular form and comprises an internal part made up of the original nicotinic acid granules and an external coating covering and protecting the former made up of the matrix which has solidified around the nicotinic acid granule. The nicotinic acid granule is thus microencapsulated in a coating of a mineral-lipid matrix.

Thanks to the specific composition of the mixture, in particular of the matrix, and to the above-described specific production process, the coating obtained is arranged continuously and uniformly around the internal part of niacin. This advantageously makes it possible to avoid (or at least greatly to limit) the presence of any surface outcrops of niacin which would constitute points permitting ready attack by gastric juices and would thus result in relatively rapid release of niacin into the organism.

The tests carried out on the granular product of the present invention, which are described in detail in the following paragraphs, have demonstrated excellent resistance to gastric attack, such as to enable gradual release of nicotinic acid along the entire gastrointestinal tract, so avoiding flushing phenomena in subjects taking the product. These results have been achieved even with granular products with a very high nicotinic acid content of greater than 40% and up to 70%.

The granular product according to the invention may be used, depending on dosage, both in the production of drugs and in the production of food supplements, which are in turn intended both for animals and for human beings.

In particular, the present granular product is preferably used as a food supplement for human beings, to be used in the preparation of solid or liquid food supplements, of beverages or solid foods, such as for example food bars, sweets, fruit juices, milk and derived products or bakery products.

In the pharmaceutical field, the microencapsulated niacin granules may be administered in the form of capsules, tablets, suspensions, etc..

### Example

40 g of appropriately hydrogenated triglycerides obtained from sunflower oil with a high C18 content, 3 g of CaSO₄*2(H₂O), 2 g of CaCO₃ and 5 g of propylene glycol distearate (PGD) as emulsifying agent were placed in a mixer. The mixture was adjusted to a temperature of 70°C, so as to melt the lipid components and form a solid suspension in the liquid phase. 50 g of pure nicotinic acid in granular form, of a size between 0.1 and 0.5 mm, were added to said first mixture, which constitutes the matrix, while the mixture was constantly agitated.

The resultant mixture was then supplied to a spraying chamber kept at a temperature of approximately -22°C where it was sprayed through a jet suited to the desired grain size, so as to obtain granules with an internal part based on niacin microencapsulated by an external coating based on a mineral-lipid matrix.

The granules taken from the spraying chamber were subjected to screening to obtain those granules of a size of between 0.25 and 0.8 mm.

### Product analysis

Three samples of the granules obtained by the above-described process were subjected to in vitro testing to determine their behaviour along the human gastrointestinal tract, simulated by three distinct phases.

In the first phase, the samples were placed in respective 100 ml conical flasks to which were added 25 ml of a 0.1 M solution of phosphate buffer at pH 6 with gentle agitation, followed by 10 ml of a 0.2 M solution of HCl, the overall pH being adjusted to 2.0. 25 mg of pepsin in solution were then added to this solution and the overall mixture was kept for 2 hours in a controlled temperature environment at 39°C.

In the second phase, a phosphate buffer and 0.6 M NaOH were added to the mixture obtained from the first phase to adjust the overall pH to 6.8, and 100 mg of pancreatin in solution were added. The samples were then incubated for 4 hours at a temperature of 39°C.

In the third phase, the overall pH of the solution was adjusted to 7.0 by addition of 1 M NaOH, after which 100 mg of lipases, obtained from pig pancreas (SIGMA L-3126), were added and the solution was incubated for 18 hours at 39°C.

At the end of each of the three phases, portions of the three samples were taken and analysed by HPLC with the aim of ascertaining their nicotinic acid concentration.

The results are shown in Table 1 below.

**Table 1**

| | Beginning | After phase 1 | After phase 2 | After phase 3 |
|---|---|---|---|---|
| Nicotinic acid concentration (%) | 53.56 | 44.32 | 34.17 | 2.72 |
| Released nicotinic acid (%) | 0 | 17.4 | 36.3 | 94.9 |

It is clear from examination of the results shown above that only very little release of niacin occurs during the time spent in the stomach and that, on the contrary, release predominantly occurs in the intestinal digestive phase due to the action of the enzymes. In this manner, virtually complete, entirely satisfactory release of the active ingredient is obtained, said release in particular being well distributed over time such that the concentration of niacin absorbed by the organism is never so high as to bring about flushing phenomena in the individual taking it.

The present invention thus solves the above-described problem with reference to the cited prior art and simultaneously provides further advantages including the production of a granular product based on microencapsulated nicotinic acid with an elevated content of active ingredient. This feature makes it possible to use a relatively limited quantity of finished granular product to administer the required quantity of nicotinic acid to the individual and proves to be particularly important when the product according to the present invention is to be used as a food supplement. It should be noted that the dosages which may be administered daily to individuals being treated with nicotinic acid are of the order of one gram.

A further advantage provided by the present invention is that of permitting safe handling of the product based on nicotinic acid without entailing special safety measures, nicotinic acid being well known to have irritant properties.

## Claims

1. A granular, gastro-resistant product based on niacin or derivatives thereof, comprising an Internal part in which said niacin or derivatives thereof is/are substantially concentrated and an external coating covering and protecting said internal part, said external coating being made up of a matrix having a total weight fraction of long-chain C16-C22 saturated fatty acids of between 40% and 95% relative to the matrix, **characterised in that** said matrix further comprises a mineral agent, dispersed into the same, in a weight fraction of between 2% and 20% relative to the matrix.

2. A product according to claim 1, in which said granular product has a size of between 0.15 and 2 millimetres.

3. A product according to claim 2, in which said granular product has a size of between 0.25 and 0.8 millimetres.

4. A product according to claim 1, 2 or 3, in which said matrix comprises a weight fraction of C18 saturated fatty acid of between 40% and 95% relative to the matrix.

5. A product according to claim 4, in which said weight fraction of C18 saturated fatty acid is between 60% and 80% relative to the matrix.

6. A product according to any of the preceding claims, in which said mineral agent is calcium sulfate dihydrate.

7. A product according to any of the preceding claims, in which said matrix comprises an effective quantity of emulsifying agent.

8. A product according to claim 7, in which said emulsifying agent is based on propylene glycol distearate.

9. A product according to any of the preceding claims, in which said niacin is present in a weight fraction of between 40% and 70%.

10. A granular, gastro-resistant product based on niacin or derivatives thereof according to any of the preceding claims, for use in the treatment of arterial hypertension or hypercholesterolaemia.

11. A process for the production of granular gastro-resistant products based on niacin or derivatives thereof, comprising the phases of:
- mixing granular material based on niacin or derivatives thereof with a matrix having a total content of long-chain C16-C22 saturated fatty acids of between 40% and 95% relative to the matrix, adjusting said mixture to a temperature such as to melt said saturated fatty acids, and
- spraying said mixture into a chamber with a temperature below the melting temperature of the matrix, such that said matrix solidifies around said granular material, so forming a product having an internal part in which said niacin or derivatives thereof is/are substantially concentrated and an external coating made up of said matrix,
**characterised in that** said matrix further comprises a mineral agent in a weight fraction of between 2% and 20% relative to the matrix.

12. A process according to claim 11, in which said granular material based on niacin has a size of between 0.1 and 1.5 millimetres.

13. A process according to claim 12, in which said granular material based on niacin has a size of between 0.1 and 0.5 millimetres.

14. A process according to claim 11, 12 or 13, in which said matrix comprises a weight fraction of C18 saturated fatty acid of between 40% and 95% relative to the matrix.

15. A process according to claim 14, in which said weight fraction of C18 saturated fatty acid is between 60% and 80% relative to the matrix.

16. A process according to any of claims 11 to 15, in which said mineral agent is calcium sulfate dihydrate.

17. A process according to any of claims 11 to 16, in which an effective quantity of emulsifying agent is provided in said matrix.

18. A process according to claim 17, in which said emulsifying agent is based on propylene glycol distearate.

19. A process according to any of claims 11 to 18, in which said niacin is mixed with said matrix in a weight fraction of between 40% and 70% relative to the complete mixture.

20. A process according to any of claims 11 to 19, in which said mixing phase is carried out at a temperature of between 60°C and 75°C.

21. A process according to any of claims 11 to 20, in which said mixture is sprayed into a chamber having a temperature of below - 5°C.

22. A process according to claim 21, in which said mixture is sprayed into a chamber having a temperature of between -20°C and -25°C.

23. Use of a granular product as defined in any of claims 1 to 10 in the production of a medicament for the treatment of arterial hypertension or hypercholesterolaemia.

24. Use of a granular product as defined in any of claims 1 to 10 as a food supplement for human beings.

## Patentansprüche

1. Granulatförmiges, magenresistentes Produkt auf der Basis von Niacin oder dessen Derivaten, umfassend einen inneren Teil, in dem das Niacin oder dessen Derivate im Wesentlichen konzentriert ist/sind, und eine äußere Beschichtung, welche den inneren Teil bedeckt und schützt, wobei die äußere Beschichtung aus einer Matrix gefertigt ist, die einen Gesamtgewichtsanteil von langkettigen gesättigten C16-C22-Fettsäuren zwischen 40 % und 95 % in Bezug auf die Matrix aufweist,
**dadurch gekennzeichnet, dass** die Matrix ferner einen mineralischen Wirkstoff, der in dieser gelöst ist, in einem Gewichtsanteil zwischen 2 % und 20 % in Bezug auf die Matrix umfasst.

2. Produkt nach Anspruch 1, wobei das granulatförmige Produkt eine Größe zwischen 0,15 und 2 Millimeter aufweist.

3. Produkt nach Anspruch 2, wobei das granulatförmige Produkt eine Größe zwischen 0,25 und 0,8 Millimeter aufweist.

4. Produkt nach Anspruch 1, 2 oder 3, wobei die Matrix einen Gewichtsanteil einer gesättigten C18-Fettsäure zwischen 40 % und 95 % in Bezug auf die Matrix umfasst.

5. Produkt nach Anspruch 4, wobei der Gewichtsanteil der gesättigten C18-Fettsäure zwischen 60 % und 80 % in Bezug auf die Matrix liegt.

6. Produkt nach einem der vorstehenden Ansprüche, wobei der mineralische Wirkstoff Kalziumsulfatdihydrat ist.

7. Produkt nach einem der vorstehenden Ansprüche, wobei die Matrix eine wirksame Menge eines emulgierenden Wirkstoffs umfasst.

8. Produkt nach Anspruch 7, wobei der emulgierende Wirkstoff auf Propylenglykol-distearat basiert.

9. Produkt nach einem der vorstehenden Ansprüche, wobei das Niacin in einem Gewichtsanteil zwischen 40 % und 70 % vorhanden ist.

10. Granulatförmiges, magenresistentes Produkt auf der Basis von Niacin oder dessen Derivaten nach einem der vorstehenden Ansprüche zur Verwendung bei der Behandlung von arterieller Hypertonie oder Hypercholesterinämie.

11. Verfahren für die Herstellung von granulatförmigen, magenresistenten Produkten auf der Basis von Niacin oder dessen Derivaten, umfassend die Phasen:
- Mischen des granulatförmigen Materials auf der Basis von Niacin oder dessen Derivaten mit einer Matrix, die einen Gesamtanteil von langkettigen gesättigten C16-C22-Fettsäuren zwischen 40 % und 95 % in Bezug auf die Matrix aufweist,
- Erwärmen der Mischung auf eine Temperatur, um die gesättigten Fettsäuren zu schmelzen,
- Sprühen der Mischung in eine Kammer mit einer Temperatur unter der Schmelztemperatur Matrix, so dass sich die Matrix um das granulatförmige Material verfestigt, um so ein Produkt mit einem inneren Teil, in dem das Niacin oder dessen Derivate im Wesentlichen konzentriert ist/sind, und einer äußeren Beschichtung aus der Matrix zu bilden,
**dadurch gekennzeichnet, dass** die Matrix ferner einen mineralischen Wirkstoff in einem Gewichtsanteil zwischen 2 % und 20 % in Bezug auf die Matrix umfasst.

12. Verfahren nach Anspruch 11, wobei das granulatförmige Material auf der Basis von Niacin eine Größe zwischen 0,1 und 1,5 Millimeter aufweist.

13. Verfahren nach Anspruch 12, wobei das granulatförmige Material auf der Basis von Niacin eine Größe zwischen 0,1 und 0,5 Millimeter aufweist.

14. Verfahren nach Anspruch 11, 12 oder 13, wobei die Matrix einen Gewichtsanteil einer gesättigten C18-Fettsäure zwischen 40 % und 95 % in Bezug auf die Matrix umfasst.

15. Verfahren nach Anspruch 14, wobei der Gewichtsanteil der gesättigten C18-Fettsäure zwischen 60 % und 80 % in Bezug auf die Matrix liegt.

16. Verfahren nach einem der Ansprüche 11 bis 15, wobei der mineralische Wirkstoff Kalziumsulfatdihydrat ist.

17. Verfahren nach einem der Ansprüche 11 bis 16, wobei eine wirksame Menge eines emulgierenden Wirkstoffs in der Matrix vorgesehen ist.

18. Verfahren nach Anspruch 17, wobei der emulgierende Wirkstoff auf Propylenglykol-distearat basiert.

19. Verfahren nach einem der Ansprüche 11 bis 18, wobei das Niacin mit der Matrix in einem Gewichtsanteil zwischen 40 % und 70 % in Bezug auf die gesamte Mischung gemischt wird.

20. Verfahren nach einem der Ansprüche 11 bis 19, wobei die Mischphase bei einer Temperatur zwischen 60°C und 75°C durchgeführt wird.

21. Verfahren nach einem der Ansprüche 11 bis 20, wobei die Mischung in eine Kammer mit einer Temperatur unter -5°C gesprüht wird.

22. Verfahren nach Anspruch 21, wobei die Mischung in eine Kammer mit einer Temperatur zwischen -20°C und -25°C gesprüht wird.

23. Verwendung eines granulatförmigen Produkts nach einem der Ansprüche 1 bis 10 bei der Herstellung eines Medikaments für die Behandlung von arterieller Hypertonie oder Hypercholesterinämie.

24. Verwendung eines granulatförmigen Produkts nach einem der Ansprüche 1 bis 10 als Nahrungsmittelzusatz für Menschen.

## Revendications

1. Produit granulaire gastro-résistant à base de niacine ou de dérivés de celle-ci comprenant une partie interne dans laquelle lesdits niacine ou dérivés de celle-ci est/sont essentiellement concentré(s) et un revêtement externe recouvrant et protégeant ladite partie interne, ledit revêtement externe étant constitué d'une matrice présentant une fraction massique totale d'acide gras saturés C16-C22 à chaîne longue entre 40 % et 95 % par rapport à la matrice, **caractérisé en ce que** ladite matrice comprend de plus un agent minéral, dispersé dans celle-ci, dans une fraction massique entre 2 % et 20 % par rapport à la matrice.

2. Produit selon la revendication 1, dans lequel ledit produit granulaire présente une taille entre 0,15 et 2 mm.

3. Produit selon la revendication 2, dans lequel ledit produit granulaire présente une taille entre 0,25 et 0,8 mm.

4. Produit selon la revendication 1, 2 ou 3, dans lequel ladite matrice comprend une fraction massique d'acide gras saturé C18 entre 40 % et 95 % par rapport à la matrice.

5. Produit selon la revendication 4, dans lequel ladite fraction massique d'acide gras saturé C18 est entre 60 % et 80 % par rapport à la matrice.

6. Produit selon l'une quelconque des revendications précédentes, dans lequel ledit agent minéral est du dihydrate de sulfate de calcium.

7. Produit selon l'une quelconque des revendications précédentes, dans lequel ladite matrice comprend une quantité effective d'agent émulsionnant.

8. Produit selon la revendication 7, dans lequel ledit agent émulsionnant est à base de distéarate de propylèneglycol.

9. Produit selon l'une quelconque des revendications précédentes, dans lequel ladite niacine est présente dans une fraction massique entre 40 % et 70 %.

10. Produit granulaire gastro-résistant à base de niacine ou de dérivés de celle-ci selon l'une quelconque des revendications précédentes pour une utilisation dans le traitement de l'hypertension artérielle ou de l'hypercholestérolémie.

11. Procédé pour la production de produits granulaires gastro-résistants à base de niacine ou de dérivés de celle-ci comprenant les phases :
- de mélange d'un matériau granulaire à base de niacine ou de dérivés de celle-ci avec une matrice présentant une teneur totale en acides gras saturés C16-C22 à chaîne longue entre 40 % et 95 % par rapport à la matrice,
- d'ajustement dudit mélange à une température telle qu'elle fait fondre lesdits acides gras saturés, et
- de pulvérisation dudit mélange dans une chambre avec une température inférieure à la température de fusion de la matrice, de telle sorte que ladite matrice se solidifie autour dudit matériau granulaire afin de former un produit présentant une partie interne dans laquelle lesdits niacine ou dérivés de celle-ci est/sont essentiellement concentré(s) et un revêtement externe constitué de ladite matrice,
**caractérisé en ce que** ladite matrice comprend de plus un agent minéral dans une fraction massique entre 2 % et 20 % par rapport à la matrice.

12. Procédé selon la revendication 11, dans lequel ledit matériau granulaire à base de niacine présente une taille entre 0,1 et 1,5 mm.

13. Procédé selon la revendication 12, dans lequel ledit matériau granulaire à base de niacine présente une taille entre 0,1 et 0,5 mm.

14. Procédé selon la revendication 11, 12 ou 13, dans lequel ladite matrice comprend une fraction massique d'acide gras saturé C18 entre 40 % et 95 % par rapport à la matrice.

15. Procédé selon la revendication 14, dans lequel ladite fraction massique d'acide saturé C18 est entre 60 % et 80 % par rapport à la matrice.

16. Procédé selon l'une quelconque des revendications 11 à 15, dans lequel ledit agent minéral est du dihydrate de sulfate de calcium.

17. Procédé selon l'une quelconque des revendications 11 à 16, dans lequel une quantité effective d'agent émulsionnant est fournie dans ladite matrice.

18. Procédé selon la revendication 17, dans lequel ledit agent émulsionnant est à base de distéarate de propylèneglycol.

19. Procédé selon l'une quelconque des revendications 11 à 18, dans lequel ladite niacine est mélangée avec ladite matrice dans une fraction massique entre 40 % et 70 % par rapport au mélange complet.

20. Procédé selon l'une quelconque des revendications 11 à 19, dans lequel ladite phase de mélange est réalisée à une température entre 60°C et 75°C.

21. Procédé selon l'une quelconque des revendications 11 à 20, dans lequel ledit mélange est pulvérisé dans une chambre ayant une température inférieure à -5°C.

22. Procédé selon la revendication 21, dans lequel ledit mélange est pulvérisé dans une chambre ayant une température entre -20°C et -25°C.

23. Utilisation d'un produit granulaire selon l'une quelconque des revendications 1 à 10 dans la production d'un médicament destiné au traitement de l'hypertension artérielle ou de l'hypercholestérolémie.

24. Utilisation d'un produit granulaire selon l'une quelconque des revendications 1 à 10 comme supplément alimentaire pour des êtres humains.
